# EUROPÄISCHE PATENTSCHRIFT

(11) **EP 0 794 431 B1**
(45) Veröffentlichungstag und Bekanntmachung des Hinweises auf die Patenterteilung: **15.10.2003**
(21) Anmeldenummer: 97103719.7
(22) Anmeldetag: 06.03.1997
(51) Int. Cl.: G01N 33/18, G01N 33/00, G01N 7/18

(54) **Verfahren zur Bestimmung des Gasverbrauchs von Materie**
Method for determining gas consumption by matter
Méthode pour déterminer la consommation de gaz par une matière

(30) Priorität: 08.03.1996 DE 19609071
(43) Veröffentlichungstag der Anmeldung: 10.09.1997
(73) Patentinhaber: WTW Wissenschaftlich-Technische Werkstätten GmbH & Co. KG, 82362 Weilheim (DE)
(72) Erfinder: Rettig, Ulrich, 82407 Wielenbach (DE); Fink, Willi, 72108 Rottenburg (DE)
(74) Vertreter: Zipse + Habersack

(56) Entgegenhaltungen:
- EP-A- 0 414 182
- US-A- 3 907 646
- US-A- 4 152 213
- US-A- 5 051 360
- US-A- 5 232 839
- KOHNE M ET AL: "DRUCKSENSOREN ERSETZEN QUECKSILBER-MANOMETER BEI DER BSB5-MESSUNG PRESSURE SENSORS SUBSTITUTE MERCURY MANO-METERS IN BOD5-MEASUREMENT" KORRESPONDENZ ABWASSER,DE,ABWASSERTECHNISCHE VEREINIGUNG, ST. AUGUSTIN, Bd. 41, Nr. 5, 1. Mai 1994 (1994-05-01), Seiten 772-774,776,777, XP000440222 ISSN: 0341-1540
- WAGNER I ET AL: "EIN QUECKSILBERFREIES BSB-MESSSYSTEM ZUR BESTIMMUNG DES BIOCHEMISCHEN SAUERSTOFFBEDARFS" KORRESPONDENZ ABWASSER,DE,ABWASSERTECHNISCHE VEREINIGUNG, ST. AUGUSTIN, Bd. 43, Nr. 4, 1. April 1996 (1996-04-01), Seiten 517-518,521-522, XP000595152 ISSN: 0341-1540
- LOGAN B E ET AL: "THE HBOD TEST: A NEW METHOD FOR DETERMINING BIOCHEMICAL OXYGEN DEMAND" WATER ENVIRONMENT RESEARCH,US,WATER ENVIRONMENT FEDERATION, ALEXANDRIA, Bd. 65, Nr. 7, 1. November 1993 (1993-11-01), Seiten 862-868, XP000415276 ISSN: 1061-4303

## Beschreibung

Die vorliegende Erfindung betrifft ein Verfahren zur Messung oder Bestimmung des Gasverbrauchs von Materie, insbesondere zur Messung des biochemischen Sauerstoffbedarfs einer in einem abgeschlossenen Behälter angeordneten Probe.

Bislang war es bei diesen Verfahren üblich, eine zu messende Probe in einem thermostatisierten bzw. klimatisierten Raum, insbesondere Inkubator anzuordnen und den Behälter offen stehen zu lassen, bis eine Adaption, d.h. eine Anpassung der Probe an die Umgebungsverhältnisse stattgefunden hat. Dann wurde der Behälter samt Meßsystem verschlossen, und es wurde mit der Messung begonnen.

Die US 5,232,839 zeigt eine Anordnung zur Bestimmung mikrobiellen Wachstums in einem Messbehälter. Die Druckschrift erkennt das Problem der Druckänderung während der Temperatureinstellung des geschlossenen System. Dieses Problem wird dort dadurch gelöst, dass während einer anfänglichen Periode der Temperatureinstellung des Messbehälters in einem Inkubator keine Messungen durchgeführt werden. Den gleichen Weg beschreitete der Aufsatz von M. Köhne et al, "Drucksensoren ersetzten Quecksilbermanometer bei der BSB 5-Messung" in Korrespondenz Abwasser, Heft 5 1994 Seiten 772-778, der mit dem Starten der Messungen nach etwa einer Stunde automatisch beginnt, nachdem sich das System eingestellt hat.

Die vorliegende Erfindung hat die Aufgabe, eine zuverlässige Fehlervermeidung bei der Messung des Gasverbrauchs von Materie, insbesondere bei der Messung des biochemischen Sauerstoffbedarfs (BSB-Messung) einer in einem abgeschlossenen Behälter angeordneten Probe unter einfacher Handhabung zu gewährleisten, insbesondere ohne daß auf den bereits im Inkubator befindlichen Behälter noch einmal zugegriffen werden muß.

Die Aufgabe wird erfindungsgemäß durch das Verfahren nach Anspruch 1 gelöst.

Erfindungsgemäß wird der Innendruck in dem Behälter von der Meßeinrichtung bereits vor dem eigentlichen Meßbeginn überwacht. In der Regel erfolgt in dieser Zeit eine Temperaturanpassung des Behälterinhalts an die Temperatur im Inkubator, d.h. die zu kalte Probe erwärmt sich auf das Temperaturniveau des Inkubators, in dem der Behälter angeordnet ist. Eine zu warme Probe kühlt sich entsprechend ab. Diese Anpassung des über der Probe befindlichen Gases ist mit einem Druckanstieg/abfall verbunden, der bei sofortigem Meßbeginn das Meßergebnis beeinflussen würde. Die Meßeinrichtung überwacht daher den Druck in dem Behälter und beginnt mit der Messung erst dann, wenn die Druckänderung aufgrund der Temperaturanpassung unbedeutend ist und daher die Meßergebnisse nicht mehr nennenswert verfälscht. Das Ende der Druckanpassung ist in der Regel dadurch erkennbar, daß die relativ hohe zeitliche Druckänderung in eine geringere zeitliche Druckänderung übergeht. Diesen Zeitpunkt kann man durch eine geeignete Wahl des vorgegebenen Wertebereichs für die zeitliche Druckänderung bestimmen und für den Start des Meßvorgangs oder für den Beginn der Auswertung der Messungen nutzen. Vorzugsweise lassen sich für unterschiedliche Proben (Füllgrade, Behältergrößen) unterschiedliche vorgegebene Wertebereiche in einem Referenzwertspeicher der Steuerung speichern, die dann eine Verwendung der Meßvorrichtung für mehrere Proben (Füllgrade, Behältergrößen) und einen probenspezifischen Start der Meßvorrichtung erlauben. Wenn daher die zeitliche Druckänderung innerhalb des vorgegebenen Wertebereichs liegt, wird die Messung gestartet. Bei einer zu kalten Probe liegt dieser Zeitpunkt dann vor, wenn kein Druckanstieg im Behälter mehr stattfindet bzw. der Druckanstieg durch biochemische Prozesse in einen Druckabfall übergeht, d.h. am Wendepunkt des Druckverlaufes.

Der Vorteil des erfindungsgemäßen Verfahrens besteht darin, daß in der Prüfphase ein zeitlicher Referenzpunkt definiert wird, welcher das Ende der Prüfphase und den Beginn der Meßphase bzw. der auswertbaren Meßergebnisse darstellt. Dieser Referenzpunkt definiert hinreichend genau den Zeitpunkt, an dem die biologische und physikalische Anpassung des Probensystems (Probe+Behälter+Meßsystem) an die Umgebung zumindest weitgehend abgeschlossen ist. Daher kann die Probe nach dem Einfüllen in den Behälter und nach dem Verbinden mit dem Meßsystem sofort verschlossen in den Inkubator gestellt werden, ohne daß auf den Behälter nachher noch einmal zugegriffen werden muß, um diesen zu schließen. Es ist selbstverständlich ebenso möglich die Druckwerte des Probensystems im Inkubator einfach, z.B. in einem Meßwertspeicher, aufzuzeichnen und die Auswertung gemäß dem erfindungsgemäßen Verfahren erst später nach Abschluß der Messung vorzunehmen. In diesem Fall wird ebenfalls der Druckverlauf gemäß Anspruch 1 ausgewertet, wobei der bei der Auswertung bestimmte Referenzzeitpunkt nicht den Meßbeginn, sondern den Beginn der auswertbaren Meßwerte definiert.

In einer vorteilhaften Weiterbildung der Erfindung kann vorgesehen sein, daß unabhängig vom Ausgangszustand des Meßsystems, d.h. unabhängig davon, ob das Meßsystem zu warm oder zu kalt ist, vor der Prüfphase eine Adaptionsphase vorgesehen wird, innerhalb der weder eine Messung noch eine Druckbewertung des Behälterdrucks stattfindet. Eine derartige Adaptionsphase erfolgt über eine Zeitspanne von 0 bis 2h und wird vorzugsweise auf einen definierten Wert, z.B. auf eine Stunde, eingestellt.

Weiterhin ist es vorteilhaft (jedoch keine Ausführungsform der vorliegenden Erfindung), wenn die Prüfphase auf einen maximalen Wert, z.B. 2,5 Stunden, einstellbar ist. Auf diese Weise wird die Prüfung der Druckänderung nach der Adaptionsphase auf eine sinnvolle Gesamtzeit limitiert.

Vorzugsweise wird die Drucküberprüfung während der Prüfphase in Zeitintervallen von 0 bis 60 min. vorgenommen, was von Vorteil ist, wenn eine batterie- oder akkumulatorbetriebene Meßvorrichtung verwendet wird. Das Zeitintervall wird auf einen geeigneten Wert eingestellt, der sich nach der üblichen Dauer der Prüfphase richtet. Es werden Zeitintervalle zwischen 15 min. und 50 min. bevorzugt. Nach jedem Zeitintervall erfolgt ein Druckvergleich mit dem letzten gespeicherten Meßwert. Ist der aktuelle Druck(Meß)wert größer als der letzte gespeicherte Druckwert, so wird der Nullpunkt des Druckmeßsystems auf den aktuellen Druckwert gesetzt und eine neues Zeitintervall wird gestartet. Erst wenn der Druck nach Ablauf des Zeitintervalls kleiner oder gleich dem letzten gesetzten Nullpunkt ist, wird das Meßsystem für die eigentliche Messung gestartet. Als Startwert für die automatische Messung wird der letzte Nullpunkt des Meßsystems herangezogen.

Während der jetzt laufenden Meßphase wird die Meßwertbildung in Zeitintervallen von 10 sek. bis 24 h vorgenommen. Die Zeitintervalle liegen bei einer batterie- oder akkumulatorbetriebenen Meßeinrichtung vorzugsweise zwischen 12 und 24 h.

In einer weiteren Ausführung mit externer Datenauswertung kann die Meßwertbildung vorzugsweise zwischen 5 und 60 min. erfolgen.

In einer Ausführung, in der das Meßsystem als reines Druckerfassungssystem ausgelegt ist, wird die Ermittlung des Startpunktes der Messung erst später nach Abschluß der gesamten Laufzeit der Druckwerterfassung durchgeführt. Hierbei wird das Prinzip der Startpunktermittlung analog zur oben dargestellten simultanen Startpunktermittlung bei der sofortigen Datenauswertung angewendet. Die Meßwertübertragung zwischen Meßsystem und Auswerteeinrichtung erfolgt entweder nach Abschluß der Messungen vom Speicher des Meßsystems Off-Line oder online über Leitung, Funk, IR, Ultraschall oder induktive Anbindung.

Das Verfahren zur Fehlervermeidung bei der Messung des Gasverbrauchs von Materie eignet sich für alle Formen von Messungen, bei der ein Stoff in einem abgeschlossenen Behälter eine Verringerung des Drucks bzw. Partialdrucks eines bestimmten Gases in dem Behälter bewirkt. Insbesondere eignet sich dieses Verfahren zur Messung des biochemischen Sauerstoffbedarfs. Bei Beginn der Messung wird der aktuelle Druckwert als Nullwert für die folgende Messung gesetzt. Vorangegangene biologische und physikalische Anpassungen des Probensystems bleiben somit bei der Auswertung der gemessenen Druckwerte für die BSB-Messung außer Betracht.

## Patentansprüche

1. Verfahren zur Messung oder Bestimmung des Gasverbrauchs einer in einem abgeschlossenen Behälter angeordneten Probe,
welcher Behälter in einem thermostatisierten bzw. klimatisierten Raum z.B. einem Klimaraum bzw. Inkubator auf eine Temperatur gebracht wird, wobei
der Behälterdruck vor dem Erhalt auswertbarer Meßwerte in einer Prüfphase überwacht wird, während welcher Prüfphase eine Temperaturanpassung der Probe an die Temperaturverhältnisse im Inkubator stattfindet, die mit einer relativ hohen zeitlichen Druckänderung im Behälterinnenraum einhergeht,
wobei ein Vergleich der zeitlichen Druckänderung im Behälterinnenraum mit einem vorbestimmten Wertebereich, entsprechend einer relativ niedrigen zeitlichen Druckänderung, erfolgt, und wobei die Meßwerte für die Bestimmung des Gasverbrauchs von einem Startzeitpunkt an ausgewertet werden, an welchem die Druckänderung innerhalb des vorbestimmten Wertebereichs liegt.

2. Verfahren nach Anspruch 1,
**dadurch gekennzeichnet,**
**daß** die Meßergebnisse erst dann für die Bestimmung des Gasverbrauchs ausgewertet werden, wenn der Druck in dem Behälter zu fallen beginnt.

3. Verfahren nach einem der vorhergehenden Ansprüche,
**dadurch gekennzeichnet,**
**daß** vor die Prüfphase eine Adaptionsphase mit einer definierten Zeitspanne gesetzt wird, in welcher noch keine Druckbewertung bzw. Druckmessung erfolgt.

4. Verfahren nach Anspruch 3,
**dadurch gekennzeichnet,**
**daß** die Zeitspanne für die Adaptionsphase zwischen 30 min und 2 h, insbesondere bei 1 h liegt.

5. Verfahren nach einem der vorhergehenden Ansprüche,
**dadurch gekennzeichnet,**
**daß** die Druckwerte während der Meßphase in bestimmten Zeitintervallen aufgezeichnet bzw. ausgewertet werden.

6. Verfahren nach einem der vorhergehenden Ansprüche,
**dadurch gekennzeichnet,**
**daß** in der Prüfphase eine Druckwertnahme/Druckprüfung in Intervallen mit einer Zeitspanne zwischen 10s bis 60 min., vorzugsweise zwischen 15 und 50 min. erfolgt.

7. Verfahren nach einem der vorhergehenden Ansprüche,
**dadurch gekennzeichnet,**
**daß** die Druckwerte des Probensystems während der Messung in einem Inkubator lediglich aufgezeichnet werden, und daß die Bestimmung des Referenzzeitpunktes, d.h. die Bestimmung der auswertbaren Druckwerte zeitlich versetzt zur Messung erfolgt.

8. Verfahren nach Anspruch 7,
**dadurch gekennzeichnet,**
**daß** die Auswertung in einer separaten Auswerteeinrichtung außerhalb des Inkubators nach Abschluß der Messungen erfolgt.

## Claims

1. A method for measuring or determining the gas consumption of a sample located in a sealed container, comprising the steps:
setting the temperature of said container in a controlled environment, e.g. in a climatic cabinet or incubator,
initiating a test phase for monitoring the pressure in said container before obtaining readings to be analyzed,
adapting the temperature of said sample to the temperature conditions in said incubator during said test phase, involving a relatively high change in pressure with time in the interior of said container,
comparing said change in pressure with time in the interior of said container to a predetermined range of values corresponding to a relatively low change in pressure with time, and
analyzing the readings for determining the gas consumption from a starting point in time at which the change in pressure is within said predetermined range of values.

2. The method as set forth in claim 1, **characterized in that** said readings for determining gas consumption are not analyzed until the pressure in said container begins to drop.

3. The method as set forth in claim 1 or 2, **characterized in that** prior to said test phase an adaptation phase is initiated with a defined time span in which no pressure analysis or pressure measurement occurs.

4. The method as set forth in claim 3, **characterized in that** said time span for said adaptation phase is in the range of 30 minutes to 2 hours, it more particularly being 1 hour.

5. The method as set forth in any of the preceding claims, **characterized in that** said pressure readings are recorded and analyzed at specific intervals during said measurement phase.

6. The method as set forth in any of the preceding claims, **characterized in that** during said test phase a pressure reading is obtained at intervals with a time span in the range of 10 seconds to 60 minutes, preferably in the range 15 to 50 minutes.

7. The method as set forth in any of the preceding claims, **characterized in that** said pressure readings of said sample sytem are merely recorded during said measurement in an incubator, and determining said reference time point, i.e. determining the pressure readings for analysis is timed staggered to the measurement.

8. The method as set forth in claim 7, **characterized in that** said analysis takes place in a separate analytical device outside of said incubator following conclusion of said measurements.

## Revendications

1. Procédé pour mesurer ou déterminer la consommation de gaz d'un échantillon disposé dans un récipient fermé, lequel récipient est amené à une température dans une chambre thermostatique ou climatisée, par exemple, une chambre climatique ou un incubateur ; la pression du récipient étant surveillée avant d'obtenir des valeurs de mesure susceptible d'être évaluées dans une phase de vérification, pendant laquelle phase de vérification on effectue un ajustement de température de l'échantillon aux conditions de températures présents dans l'incubateur, lequel ajustement s'accompagne d'une modification temporelle de la pression relativement élevée dans la chambre intérieure du récipient ; une comparaison de la modification temporelle de pression dans la chambre intérieure du récipient étant effectué avec une zone de valeurs prédéterminée correspondant à une modification temporelle de la pression relativement faible et les valeurs de mesure pour déterminer la consommation de gaz à partir d'un point de départ étant évaluées où se situe la modification de pression de la zone de valeur prédéterminée.

2. Procédé selon la revendication 2, **caractérisé en ce que** les résultats des mesures seront évalués seulement lors de la détermination de la consommation de gaz lorsque la pression commence à tomber dans le récipient.

3. Procédé selon une des revendications précédentes, **caractérisé en ce qu'**avant la phase de vérification on introduit une phase d'adaptation avec un laps de temps défini dans lequel n'apparaît encore aucune évaluation de la pression ou aucune mesure de pression.

4. Procédé selon la revendication 3, **caractérisé en ce que** le laps de temps se trouve, pour la phase d'adaptation, entre 30 minutes et 2 heures, notamment à 1 heure.

5. Procédé selon une des revendications précédentes, **caractérisé en ce que** les valeurs de pression sont enregistrées ou évaluées pendant la phase de mesure à des intervalles de temps déterminés.

6. Procédé selon une des revendications précédentes, **caractérisé en ce qu'**on réalise pendant la phase de vérification, une détection / vérification de la pression selon des intervalles avec un laps de temps compris entre 10 secondes à 60 minutes, de préférence, entre 15 et 50 minutes.

7. Procédé selon une des revendications précédentes, **caractérisé en ce que** les valeurs de pression du systèmes d'échantillons sont enregistrées uniquement pendant la prise de mesure dans un incubateur et **en ce que** la détermination du point de référence , c'est-à-dire, la détermination des valeurs de pression susceptibles d'être évaluées est décalée dans le temps pour être mesurés.

8. Procédé selon la revendication 7, **caractérisé en ce que** l'évaluation a lieu dans un dispositif d'évaluation séparé à l'extérieur de l'incubateur après la fin des prises de mesure.
